**Europäisches Patentamt**

**European Patent Office**

**Office européen des brevets**

(11) Veröffentlichungsnummer: **0 092 801**
**B1**

(12) **EUROPÄISCHE PATENTSCHRIFT**

(45) Veröffentlichungstag der Patentschrift:
**08.01.86**

(21) Anmeldenummer: **83103887.2**

(22) Anmeldetag: **20.04.83**

(51) Int. Cl.⁴: **G 01 N 33/68,** G 01 N 33/531,
G 01 N 33/536, G 01 N 33/92,
C 12 Q 1/60

(54) Verfahren zur Bestimmung der Low Density Lipoproteine (LDL) und Reagenz zu seiner Durchführung.

(30) Priorität: **23.04.82 DE 3215310**

(43) Veröffentlichungstag der Anmeldung:
**02.11.83 Patentblatt 83/44**

(45) Bekanntmachung des Hinweises auf die Patenterteilung:
**08.01.86 Patentblatt 86/2**

(84) Benannte Vertragsstaaten:
**AT BE CH DE FR GB IT LI LU NL SE**

(56) Entgegenhaltungen:
DE - A - 2 600 664
DE - A - 2 857 710
DE - A - 3 009 037
DE - B - 2 612 725
FR - A - 2 455 743
GB - A - 1 557 807
US - A - 4 039 285

(73) Patentinhaber: **BOEHRINGER MANNHEIM GMBH,
Patentabteilung, Abt. E Sandhofer
Strasse 112-132 Postfach 31 01 20,
D-6800 Mannheim 31 Waldhof (DE)**

(72) Erfinder: **Ziegenhorn, Joachim, Dr., Ina-Seidel-Weg 1,
D-8130 Starnberg (DE)**
Erfinder: **Schiefer, Sigbert, Dr., Moosstrasse 3,
D-8121 Pähl (DE)**
Erfinder: **Dräger, Brigitte, Dr., Hofmairstrasse 15,
D-8132 Tutzing (DE)**

(74) Vertreter: **Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren und ein Reagenz zur Bestimmung der Low Density Lipoproteine (LDL).

Die Bestimmung der LDL-Fraktion (Low Density Lipoproteine), auch β-Lipoproteinfraktion genannt, hat für die differenzierte Diagnose einer Lipidstoffwechselstörung erhebliche Bedeutung erlangt.

Hypercholesterinämie und Hypertriglyceridämie begünstigen die Entstehung der Atherosklerose und des Herzinfarkts. Die Bestimmung von Cholesterin und Triglyceriden im Serum gehören daher zu dem am häufigsten durchgeführten Tests im klinisch-chemischen Routinelabor.

Zahlreiche Untersuchungen zum Fettstoffwechsel kommen zu dem Schluss, dass das individuelle Coronarrisiko besser erfasst werden kann, wenn nicht nur die Veränderung im Triglycerid- und Cholesterinspiegel bestimmt, sondern die zugrundeliegenden pathologischen Verschiebungen im Lipoproteinmuster ermittelt werden [«Münch. med. Wschr.», *121* (1979) 1639].

Die bekannten Plasmalipoproteine enthalten einen unterschiedlich hohen Anteil an Protein (Apolipoproteine), Phospholipiden, Cholesterin und Triglyceriden. Sie lassen sich aufgrund ihres Verhaltens (unterschiedliche Dichte) in der analytischen Ultrazentrifuge und aufgrund ihrer unterschiedlichen Wanderungsgeschwindigkeit in der Gel-Elektrophorese in vier verschiedene Klassen unterteilen:

Chylomikronen

Prä-β-Lipoprotein = VLDL (Very Low Density Lipoprotein)

β-Lipoprotein = LDL (Low Density Lipoprotein)

α-Lipoprotein = HDL (High Density Lipoprotein)

Die Untersuchung der Funktion der Lipoproteine ergab, das LDL innerhalb der Lipoproteine die entscheidende atherogene Komponente darstellt, bei deren Erhöhung im Blut ein gesteigertes Risiko für eine coronare Herzkrankheit vorliegt. Die frühzeitige Erkennung und Bekämpfung dieses Zustands ist von grosser Bedeutung. Es besteht daher ein Bedarf nach einem praktikablen Verfahren zur quantitativen Bestimmung der LDL-Konzentration im Serum und Plasma.

Bisher wurden für die Bestimmung des LDL-Cholesterinwertes im wesentlichen drei Methoden eingesetzt, die jedoch Nachteile besitzen:

1. Ultrazentrifugation

Dieses Verfahren ist für ein Routinelabor nicht geeignet, da man hierzu eine spezielle Geräteausrüstung benötigt und die Durchführung eine äusserst sorgfältige Arbeitstechnik und einen sehr hohen Zeitaufwand (mehrtägiges Zentrifugieren) in einer Ultrazentrifuge erfordert. Somit blieb dieses Analysenverfahren bisher nur dem Labor der forschenden Medizin vorbehalten.

2. Elektrophoretische Trennung mit anschliessender Visualisierung der Lipoproteinbanden durch Polyanionenfällung und Umrechnung der Trübungseinheiten in Cholesterinwerte

Dieses Verfahren jedoch ist zeitaufwendig und erfordert eine eigene Elektrophorese-Apparatur sowie ein Densitometer zur Auswertung [«Lab. Med.», *1* (1977) 145].

3. Bestimmung des LDL-Cholesterin-Wertes über die Friedewald-Formel [«Clin. Chem.», *18* (1972) 499]

Für die Errechnung des LDL-Cholesterin-Wertes nach der Friedewald-Formel ist die Bestimmung von 3 Parametern notwendig: Cholesterin-, HDL-Cholesterin- und Triglyceridwert der Probe. Die Methode ist damit nicht ausreichend praktikabel. Ausserdem gilt diese Näherungsformel nur für chylomikronenfreie Proben und Proben mit Triglyceridwerten unter 400 mg/dl.

4. Fällungsreaktionen

Ein Verfahren, bei welchem LDL mit Hilfe eines Lectins präzipitiert wird, ist in der DE-OS Nr. 2857710 beschrieben. Jedoch kann bei dieser Methode der Wert für LDL-Cholesterin erst nach Wiederauflösen des Präzipitats bzw. nur durch Differenzbildung der Cholesterinwerte vor und nach Präzipitation ermittelt werden. Dies stellt einen erheblichen Nachteil dar.

Eine Präzipitationsmethode für Lipoproteine, bei welcher LDL im Überstand der Fällung verbleibt, wird in der DE-PS Nr. 2600664 beschrieben. Die Methode ist jedoch für die Anwendung als Routinebestimmung nicht ausreichend praktikabel, da für die Ausfällung der Lipoproteine 2 Arbeitsschritte erforderlich sind (Zugabe zweier unterschiedlicher Agentien — Polyethylenimin und ein Kationenaustauscher — verbunden mit einer dazwischenliegenden Inkubationsphase).

Aus der FR-A Nr. 2455743 ist ein ELISA-Verfahren zur Bestimmung der Serumapoproteine bekannt, bei dem eine bestimmte Menge eines immobilisierten spezifischen Anti-Apoprotein-Antikörpers mit der Probe und einem Lipoprotein-Enzym-Konjugat umgesetzt und nach Phasentrennung die trägerfixierte Enzymaktivität gemessen wird. Bei Verwendung von LDL-Antikörpern sollen damit auch LDL bestimmt werden können. Dieses Verfahren ist aufwendig, da es die Trägerfixierung der Antikörper und die Herstellung eines Enzymkonjugates erfordert und eine Phasentrennung umfasst.

Es besteht daher ein Bedarf nach einem einfachen Verfahren und Reagenz mit hoher Praktikabilität und grosser Treffsicherheit für die Bestimmung von LDL-Lipoprotein.

Diese Aufgabe wird erfindungsgemäss gelöst durch ein Verfahren zur Bestimmung von Low Density Lipoprotein (LDL) in Körperflüssigkeiten, welches dadurch gekennzeichnet ist, dass man der zu untersuchenden Probe High-Density-Lipoprotein (HDL)-Antikörper, die mit gereinigter vollständiger HDL-Fraktion als Immunogen erhalten worden sind, zusetzt, gebildetes Unlösliches abtrennt und im Überstand das LDL oder eine seiner Komponenten bestimmt.

Die Erfindung beruht auf der überraschenden Feststellung, dass mit HDL-Antikörpern sämtliche die LDL-Bestimmung störenden Lipoproteinfraktionen, neben HDL selbst insbesondere VLDL und die Chylomikronen, ausgefällt werden, LDL selbst jedoch von der Fällung nicht erfasst wird. Dies ist insbesondere deshalb überraschend, als sowohl HDL als auch LDL, VLDL und Chylomikronen einen Proteinanteil aufweisen, der die gleichen Apolipoproteine enthält, wenn auch in sehr unterschiedlichen Konzentrationen. Es war daher nicht vorhersehbar, dass Antikörper gegen HDL nicht nur HDL sondern auch VLDL und Chylomikronen quantitativ ausfällen, ohne dabei LDL zu beeinflussen.

Die Fällung von VLDL und Chylomikronen kann beschleunigt werden, indem man zusätzlich bekannte Fällungsmittel für diese Substanzen verwendet.

Die im Überstand des Reagenz verbliebene LDL-Fraktion kann dann nach den hierfür üblichen Methoden bestimmt werden. Bevorzugt erfolgt die Bestimmung des darin enthaltenen gebundenen Cholesterins unter Anwendung der hierfür bekannten Methoden. So kann die Bestimmung beispielsweise durch Verseifung mit alkoholischer Kalilauge und chemische Bestimmung nach Liebermann-Burchard durchgeführt werden. Bevorzugt erfolgt jedoch eine enzymatische Bestimmung unter Verwendung von Cholesterinoxidase und einem Cholesterinester spaltenden Enzym oder Enzymsystem, wie insbesondere Cholesterinesterase. Bei Anwendung der letzteren Methode kann verbrauchter Sauerstoff, gebildetes Cholestenon oder, am meisten bevorzugt, gebildetes $H_2O_2$ nach den dem Fachmann hierfür bekannten Methoden bestimmt werden. Da die Bestimmung des gebundenen Cholesterins dem Fachmann bekannt ist, braucht hierauf im vorliegenden Zusammenhang nicht näher eingegangen zu werden. Es sei jedoch bemerkt, dass im Rahmen des erfindungsgemässen Verfahrens durch die Entfernung der VLDL- und Chylomikronen-Fraktionen das Auftreten von Trübungen verhindert wird, welche eine optische Messung von Cholestenon oder $H_2O_2$ im Rahmen von Farbsreaktionen stören könnten. Das Verfahren eignet sich daher in besonderem Masse in Verbindung mit einer colorimetrischen Cholesterinbestimmungsmethode.

Es ist jedoch auch möglich, anstelle des in der LDL-Fraktion enthaltenen Cholesterins bzw. anderer LDL-Komponenten wie Apolipoprotein B, Phospholipide und Triglyceride die LDL-Fraktion selbst zu bestimmen, wobei ebenfalls an sich bekannte Methoden angewendet werden können. Beispielsweise genannt sei die nephelometrische Bestimmung oder die in der DE-OS Nr. 3007764 beschriebene turbidimetrische Bestimmung.

Die HDL-Antikörper werden im Rahmen der Erfindung entweder in Form eines HDL-Antiserums, als entfettetes HDL-Antiserum oder in Form von gereinigten HDL-Antikörperfraktionen verwendet. Es ist auch möglich, HDL-Antikörperfragmente, beispielsweise Fab, $Fab_2$ und Fab'-Fragmente zu verwenden.

Die Herstellung der erfindungsgemäss verwendeten Antikörper erfolgt mit Verwendung von reinem HDL als Immunogen. Für die Antikörpergewinnung können die üblicherweise verwendeten Tierspezies herangezogen werden. Bevorzugt werden Schaf und Kaninchen. Für die Antikörpergewinnung können aber ausser den schon erwähnten Tieren bzw. vergleichbaren Lebewesen auch Zellkulturen verwendet werden.

Die Abtrennung der beim Zusatz der HDL-Antikörper gebildeten Immunaggregate kann ebenfalls nach üblichen Methoden erfolgen. Werden lösliche HDL-Antikörper verwendet, so erfolgt die Abtrennung zweckmässig durch Zentrifugation. Werden immobilisierte, trägergebundene HDL-Antikörper eingesetzt, so lassen sich die Immunaggregate durch einfache Trennung der flüssigen Phase von der kompakten festen Phase, beispielsweise einem mit Antikörper beschichteten Festkörper, trennen. Erfindungsgemäss verwendet man als Immunogen gereinigte vollständige HDL-Fraktionen. Die Reinigung erfolgt zweckmässig in an sich bekannter Weise durch Isolierung in der Ultrazentrifuge. Zusätzlich kann ggf. eine weitere Reinigung über z. B. immobilisiertes Concanavalin A nach den Methoden der Affinitätschromatographie oder der Elektrophorese vorgenommen werden. Diese Methoden sind dem Fachmann bekannt und brauchen hier nicht näher beschrieben zu werden.

Ein weiterer Gegenstand der Erfindung ist ein Reagenz zur Bestimmung der LDL-Fraktion von Körperflüssigkeiten, welches durch einen Gehalt an HDL-Antikörpern, die mit gereinigter vollständiger HDL-Fraktion als Immunogen erhalten worden sind, und eines Reagenz zur Bestimmung von Cholesterin gekennzeichnet ist.

Ein bevorzugtes Reagenz zur Bestimmung von Cholesterin enthält Cholesterinoxidase, ein Cholesterinester spaltendes Enzym oder Enzymsystem, ein System zur Bestimmung von $H_2O_2$ und ein oberflächenaktives Mittel.

Gemäss einer besonders bevorzugten Ausführungsform des vorstehend erwähnten Reagenz besteht dieses im wesentlichen aus HDL-Antikörper, Cholesterinoxidase, Cholesterinesterase, Peroxidase, 3,4-Dichlorphenol, Phenol, 4-Aminophenazon, einem nichtionischen Detergenz, Magnesiumaspartat und Puffer pH 7 bis 8,5.

Das erfindungsgemässe Reagenz enthält den Antikörper zweckmässig im Konzentrationsbereich von $10^{-7}$ bis $10^{-3}$ mol/l (bzw. Kilo Festkörper), bezogen auf die Bestimmungslösung. Der Antikörper kann in fester Form, vorzugsweise lyophilisiert, oder in Form einer Lösung vorliegen. Als Lösungsmittel können Wasser, physiologischen Kochsalzlösung, Serummilieu, Puffer wie z. B. 0,01 bis 0,5 m Trispuffer, pH 7 bis 8,5 oder 0,005 bis 0,1 m Phosphatpuffer, pH 6,5 bis 8,5, ggf. mit Kochsalzzusatz in der Grössenordnung physiologischer Kochsalzlösungen beispielsweise verwendet werden. Wird der Antikörper in immobilisierter Form verwendet, so sind Beispiele für geeignete Trägersubstanzen Polysaccharide wie Cellulose, Dextran, Stärke und deren Derivate, Silikate, Polyamide, Kollagen, Latex, Aluminiumoxid, Rinderserumalbumin und ähnliche Trägersubstanzen. Der Antikörper kann auch an der Oberfläche von Testbehältern, wie Kunststoffreagenz-Gläsern, gebunden vorliegen.

Das Reagenz kann ausserdem bekannte Fällungsmittel für VLDL und Chylomikronen enthalten.

Ein wesentlicher Vorteil des erfindungsgemässen Verfahrens liegt darin, dass sich nach Zugabe eines einzigen Reagenzes die Lipoproteinklassen — mit Ausnahme von LDL — aus der Probe entfernen lassen und der diagnostisch bedeutsame LDL-Gehalt oder der Cholesteringehalt der LDL-Fraktion anschliessend ohne weitere Probenvorbehandlung einer direkten Messung zugänglich ist. Vorteilhaft ist weiter, dass die in der Probe Trübungen verursachenden triglyceridreichen Lipoproteinklassen entfernt werden, so dass zur anschliessenden LDL- oder Cholesterinbestimmung eine klare Probe zur Verfügung steht.

Die folgenden Beispiele erläutern die Erfindung anhand einer bevorzugten Ausführungsform.

*Beispiel 1:*

A) *Herstellung von gereinigter HDL*

Nach Abtrennung von VLDL und LDL in der Ultrazentrifuge wird eine eng geschnittene HDL-Fraktion (d 1,080 bis 1,210) in der Ultrazentrifuge isoliert, wie bei V.P. Skipski: Lipid composition of lipoproteins in normal and diseased states, in: Blood Lipids and Lipoproteins: Quantitation, Composition and Metabolism. Hrsg.: Nelson, Wiley, New York, 1971, 471-583 beschrieben. Die Fraktion wird anschliessend noch zweimal bei den Dichten 1,080 und 1,210 sedimentiert bzw. flotiert.

Die HDL-Fraktion wird über immobilisiertes Concanavalin A [(1974) «Febs Lett.», *91*, 174-198] affinitätschromatographisch oder mittels Geon-Pevicon-Block-Elektrophorese nach R.W. Mahley, K.S. Holcombe (1977), «J. Lipid. Res.», *18*, 314-324, elektrophoretisch gereinigt.

B) *Herstellung des Antiserums*

Tierspecies: Schaf oder Kaninchen.

Unter Verwendung des nach A erhaltenen Immunogens wird folgendes Immunisierungsschema angewendet:

| Tag | Applikation | Immunogenmenge |
|---|---|---|
| 0 | intradermal | 1 mg Protein (HDL) emulgiert in Freund Adjuvans |
| 7 | intramuskulär | » |
| 14 | subcutan | » |
| 30 | intramuskulär | » |
| 60 | subcutan | » |
| Alle weiteren 30 Tage | subcutan | » |
| Die erste Probeblutung wird nach 45 Tagen vorgenommen. | | |

C) 50 µl Serum werden mit 150 µl eines nach B hergestellten Antiserums vermischt. Nach 30 min Inkubation bei Raumtemperatur wird der entstandene Niederschlag abzentrifugiert (2 min bei 10 000 g)

50 µl des klaren Präzipitationsüberstands werden mit 2 ml eines Reagenzes versetzt, das 0,1 mol/l Tris-Puffer, pH 7,7; 0,05 mol/l Magnesiumaspartat; 1 mmol/l 4-Aminophenazon, 6 mmol/l Phenol, 4 mmol/l 3,4-Dichlorphenol, 0,3% Fettalkoholpolyglycoläther, 400 U/l

Cholesterinesterase, 250 U/l Cholesterinoxidase und 200 U/l Peroxidase enthält.

Nach 20 min Inkubationszeit bei Raumtemperatur wird die Extinktion der Probe gegen den Reagenzienleerwert gemessen (der Reagenzienleerwert enthält 50 µl des Antiserums und berücksichtigt den Cholesteringehalt des Antiserums).

$$\Delta E = \Delta E_{Probe} - \Delta E_{Reagenzienleerwert}$$

LDL-Cholesterin (mg/dl) = 1,385 × $\Delta E$

| Serum | Aussehen | Cholesterin (mg/dl) | Triglyceride (mg/dl) | LDL-Cholesterin | |
|---|---|---|---|---|---|
| | | | | NIH-Verfahren* (mg/dl) | Immunologische Präzipitation (mg/dl) |
| 1 | trüb | 353 | 503 | 234 | 238 |
| 2 | klar | 638 | 591 | 510 | 495 |
| 3 | klar | 350 | 153 | 237 | 231 |
| 4 | trüb | 195 | 575 | 102 | 95 |
| 5 | enthält Chylomikronen | 231 | 379 | 149 | 130 |
| * Als Referenzmethode dient das NIH-Verfahren (nach Abtrennung von VLDL und Chylomikronen in der Ultrazentrifuge wird LDL präzipitiert; aus der Differenz der Chorinwerte vor und nach Fällung erhält man den Wert für LDL-Cholesterin). | | | | | |
| Manual of Laboratory Operations, Lipid Research Clinics Program, Lipid and Lipoprotein Analysis, DHEW Publication No. 65-628. | | | | | |

*Beispiel 2:*

Eine Lösung von je 50 µl VLDL, HDL, LDL bzw. Chylomikronen wurden mit 150 µl eines Kaninchen-Antiserums, das mit HDL als Immunogen erzeugt wurde, vermischt. Nach Zentrifugation wurde der Cholesteringehalt wie in Beispiel 1 beschrieben, im Überstand bestimmt.

| Probe | Cholesteringehalt der Probe | |
|---|---|---|
| | vor Fällung (mg/dl) | nach Fällung (mg/dl) |
| Chylomikronen | 61,4 | 2,6 |
| VLDL | 23,2 | 2,8 |
| LDL | 177,0 | 165,0 |
| HDL | 50,9 | 0,0 |

## Patentansprüche

1. Verfahren zur Bestimmung der Low Density Lipoproteine (LDL) in Körperflüssigkeiten, dadurch gekennzeichnet, dass man der zu untersuchenden Probe High-Density-Lipoprotein (HDL)-Antikörper, die mit gereinigter vollständiger HDL-Fraktion als Immunogen erhalten worden sind, zusetzt, gebildetes Unlösliches abtrennt und im Überstand das LDL oder eine seiner Komponenten bestimmt.

2. Verfahren nach Anspruch 1, dadurch gekennzeichnet, dass man als LDL-Komponente Cholesterin bestimmt.

3. Verfahren nach einem der beiden Ansprüche 1 oder 2, dadurch gekennzeichnet, dass man Antikörper in Form von HDL-Antiserum, entfettetem HDL-Antiserum oder als gereinigte Antikörperfraktion zusetzt.

4. Verfahren nach einem der Ansprüche 1 bis 3, dadurch gekennzeichnet, dass man Fragmente von HDL-Antikörpern zusetzt.

5. Verfahren nach einem der vorhergehenden Ansprüche, dadurch gekennzeichnet, dass man Antikörper vom Schaf oder Kaninchen verwendet.

6. Reagenzzusatz zur Bestimmung der LDL-Fraktion in Körperflüssigkeiten, dadurch gekennzeichnet, dass es HDL-Antikörper, die mit gereinigter vollständiger HDL-Fraktion als Immunogen erhalten worden sind und ein Reagenz zur Bestimmung von Cholesterin enthält.

7. Reagenzzusatz nach Anspruch 6, dadurch gekennzeichnet, dass das Reagenz zur Bestimmung von Cholesterin Cholesterinoxidase, ein Cholesterinester spaltendes Enzym oder Enzymsystem, ein System zur Bestimmung von $H_2O_2$ und ein oberflächenaktives Mittel enthält.

8. Reagenzzusatz nach Anspruch 7, dadurch gekennzeichnet, dass es im wesentlichen aus HDL-Antikörper, Cholesterinoxidase, Cholesterinesterase, Peroxidase, 3,4-Dichlorphenol, Phenol, 4-Aminophenazon, einem nichtionischen Detergenz, Magnesiumaspartat und Puffer pH 7 bis 8,5 besteht.

9. Reagenzzusatz nach einem der Ansprüche 6 bis 8, dadurch gekennzeichnet, dass es die HDL-Antikörper in immobilisierter Form enthält.

## Revendications

1. Procédé pour le dosage des lipoprotéines de faible densité (LDL) dans des fluides corporels, caractérisé par le fait que l'on ajoute à l'échantillon devant être examiné des anticorps lipoprotéiniques de haute densité (HDL) qui ont été obtenus avec une fraction purifiée complète (HDL) en tant qu'immunogène, on sépare l'insoluble formé et on dose dans le surnageant le (LDL) ou l'un de ses constituants.

2. Procédé selon la revendication 1, caractérisé par le fait qu'on dose du cholestérol en tant que constituant (LDL).

3. Procédé selon l'une des revendications 1 ou 2, caractérisé par le fait que l'on ajoute des anticorps sous forme d'antisérum (HDL), d'antisérum (HDL) débarrassé des graisses ou en tant que fraction d'anticorps purifiée.

4. Procédé selon l'une des revendications 1 à 3, caractérisé par le fait que l'on ajoute des fragments d'anticorps (HDL).

5. Procédé selon l'une des revendications précédentes, caractérisé par le fait que l'on utilise des anticorps de mouton ou de lapin.

6. Additif pour réactif pour le dosage de la fraction (LDL) dans des fluides corporels, caractérisé par le fait qu'il contient des anticorps (HDL) qui ont été obtenus avec la fraction (HDL) purifiée complète en tant qu'immunogène, ainsi qu'un réactif pour le dosage du cholestérol.

7. Additif pour réactif selon la revendication 6, caractérisé par le fait que le réactif pour le dosage du cholestérol contient de la cholestérol-oxydase, une enzyme ou un système enzymatique fractionnant les esters de cholestérol, un système pour le dosage de $H_2O_2$ et un agent surfactif.

8. Additif pour réactif selon la revendication 7, caractérisé par le fait qu'il est constitué essentiellement d'anticorps (HDL), de cholestérol-oxydase, de cholestérol-estérase, de peroxydase, de 3,4-dichlorophénol, de phénol, de 4-aminophénazone, d'un détergent non ionique, d'un aspartate de magnésium et de tampon pH 7 à 8,5.

9. Additif pour réactif selon l'une des revendications 6 à 8, caractérisé par le fait qu'il contient les anticorps (HDL) sous forme immobilisée.

## Claims

1. Process for the determination of low density lipoproteine (LDL) in body fluids, characterised in that one adds to the high density lipoproteins (HDL) antibodies, which have been obtained with purified complete (HDL) fraction as immunogen, separates off insolubles formed and determines the (LDL) or one of its components in the supernatant.

2. Process according to Claim 1, characterised in that one determines cholesterol as (LDL) component.

3. Process according to Claim 1 or 2, characterised in that one adds antibodies in the form of HDL antiserum, defatted (HDL) antiserum or as purified antibody fraction.

4. Process according to one of Claims 1 to 3, characterised in that one adds fragments of (HDL) antibodies.

5. Process according to one of the preceding claims, characterised in that one uses antibodies from sheep or rabbits.

6. Reagent addition for the determination of the (LDL) fraction in body fluids, characterised in that it contains (HDL) antibodies, which have been obtained with purified complete (HDL) fraction as immunogen, and a reagent for the determination of cholesterol.

7. Reagent addition according to Claim 6, characterised in that the reagent for the determination of cholesterol contains cholesterol oxidase, a cholesterol ester-splitting enzyme or enzyme system, a system for the determination of $H_2O_2$ and a surface-active agent.

8. Reagent addition according to Claim 7, characterised in that it consists essentially of (HDL) antibodies, cholesterol oxidase, cholesterol esterase, peroxidase, 3,4-dichlorophenol, 4-aminophenazone, a non-ionic detergent, magnesium aspartate and buffer pH 7 to 8.5.

9. Reagent according to one of Claims 6 to 8, characterised in that it contains the (HDL) antibodies in immobilised form.